# EUROPEAN PATENT APPLICATION

(11) **EP 4 616 901 A1**
(43) Date of publication of application: **17.09.2025**
(21) Application number: 23902812.9
(22) Date of filing: 15.12.2023
(51) Int. Cl.: A61N 5/10

(54) **SUPPORT APPARATUS, SUPPORT APPARATUS POSITIONING METHOD, AND RADIATION THERAPY SYSTEM**

(30) Priority: 17.12.2022 CN 202211628120; 11.12.2023 CN 202311694741
(71) Applicant: Neuboron Therapy System Ltd., Xiamen, Fujian 361026 (CN)
(72) Inventor: GONG, Qiuping, Nanjing, Jiangsu 211112 (CN); LIU, Yuanhao, Nanjing, Jiangsu 211112 (CN); SHU, Diyun, Nanjing, Jiangsu 211112 (CN)
(74) Representative: Gunzelmann, Rainer
(86) International application number: PCT/CN2023/139007
(87) International publication number: WO 2024/125619

(57) **Abstract**

Provided are a supporting device, a method for positioning a supporting device, and a radiotherapy system. The supporting device includes a main plate and an adapting assembly, where the main plate is used for at least partially bearing an irradiated body, the main plate defines an extension direction, and the main plate includes a supporting surface arranged in the extension direction, a side surface connected to the supporting surface, and a back surface arranged opposite the supporting surface; and the adapting assembly is arranged on the back surface and/or the side surface of the main plate and used for connecting the main plate to a predetermined location. The main plate is used for being loaded onto a bearing device for bearing the irradiated body, and the main plate can be matched and positioned to bearing devices in different scenes or in different treatment phases by the adapting assembly. A worker is only required to position the main plate to a to-be-used bearing device. A diseased part supporting assembly is mounted by additionally arranging the main plate to the bearing device, such that a problem that it is difficult to mount the diseased part supporting assembly to an existing bearing device in the past is resolved, and no complex mounting structure is required to be additionally arranged on the bearing device.

## Description

### TECHNICAL FIELD

The disclosure relates to the technical field of medical apparatuses, and particularly relates to a supporting device, a method for positioning a supporting device, and a radiotherapy system.

### BACKGROUND

As atomic science develops, radiotherapy using Cobalt-60, a linear accelerator, or an electronic beam, etc. has become a principal means for treating cancer. However, through conventional photon or electron therapy limited to a physical condition of a reflected ray, a large amount of normal tissue in a path of a beam will be damaged while a tumor cell is killed. In addition, since tumor cells are differently sensitive to radioactive rays, the conventional radiotherapy generally has a poor effect on a malignant tumor having high radiation resistance.

In order to reduce radiation injury to normal tissue around a tumor, targeted therapy in chemotherapy is applied to the radiotherapy. For tumor cells having high radiation resistance, radiation sources having high relative biological effects, for example, proton therapy, heavy ion therapy, and neutron capture therapy, are positively developed at present. Specifically, the neutron capture therapy combines a relative biological effect and radiotherapy. For example, boron neutron capture therapy provides, through specific aggregation of a boron-containing medicine in a tumor cell and in combination with precise neutron beam radiation, a better cancer therapy option than the conventional radiotherapy.

A radiotherapy apparatus is mainly used for radiotherapy of a malignant tumor. The radiotherapy aims to irradiate a target region to the maximum extent to avoid excessive irradiation on surrounding normal tissue. An irradiated body will be treated in various poses in an existing radiotherapy apparatus. A bearing device such as a treatment couch is the most vital component for supporting and positioning the irradiated body, and a diseased part of the irradiated body is supported mainly by a diseased part supporting device. However, during actual treatment, for example, when a diseased part of a patient is positioned to an effective treatment location, there are still some problems such as how to rapidly and accurately mount a device for supporting a head of a patient to bearing devices of different models, in different scenes, or in different treatment phases, and how to shorten preparation time and simplify preparation work for positioning. Especially, in a situation that an irradiation location of the patient is required to be adjusted or changed, or preparation or treatment is required to be performed in different phases and scenes, when the irradiation location is adjusted, it is difficult to resolve problems of how to reduce an adverse impact that may be imposed on a treatment effect during movement or pose adjustment of the patient, and how to ensure that the patient is treated at a location predetermined according to a treatment plan.

Based on this, a supporting device, a method for positioning a supporting device, and a radiotherapy system are provided to resolve the above problems.

### SUMMARY

To resolve the above problems, a first aspect of the disclosure provides a supporting device. The supporting device includes a main plate and an adapting assembly. The main plate is used for at least partially bearing an irradiated body. The main plate defines an extension direction. The main plate includes a supporting surface arranged in the extension direction, a side surface connected to the supporting surface, and a back surface arranged opposite the supporting surface. The adapting assembly is arranged on the back surface and/or the side surface of the main plate and used for connecting the main plate to a predetermined location. The main plate is used for being loaded onto a bearing device for bearing the irradiated body. The main plate can be matched and positioned to bearing devices in different scenes or in different treatment phases by the adapting assembly. That is, in a plurality of scenes, the main plate can be mounted by the adapting assembly, and a worker is only required to position the main plate to a to-be-used bearing device. When the irradiated body is required to be moved for scene switching or is required to be supported and positioned in different phases, by mounting and positioning the supporting device, it can be still ensured that the irradiated body maintains a predetermined pose when being positioned again and is not affected by movement and adjustment of the irradiated body. The irradiated body can be separated from the supporting device during movement such that no additional constraint and burden exist. Further, a diseased part supporting assembly is mounted by additionally arranging the main plate to the bearing device, such that universality of the main plate is extremely improved. A problem that it is difficult to mount the diseased part supporting assembly to an existing bearing device in the past is resolved. Moreover, no complex mounting structure is required to be additionally arranged on the bearing device. Thus, the irradiated body and a diseased part thereof can be rapidly and reliably supported.

According to the supporting device provided in embodiments of the disclosure, by additionally arranging a main plate on a bearing device for bearing an irradiated body, a problem that it is difficult to mount a diseased part supporting assembly to an existing bearing device can be resolved. The existing bearing device is not required to be redesigned or no complex mounting structure is required to be additionally arranged on the bearing device. Moreover, the main plate can be rapidly and reliably positioned and mounted to a predetermined location on the bearing device by an adapting assembly such that the main plate and the bearing device can be positioned at relative locations. Subsequently, after the irradiated body is transferred in different scenes, the main plate and the irradiated body can be rapidly and accurately positioned again according to the locations. Thus, it is ensured that the main plate and the irradiated body are located at the same locations as far as possible in different scenes. Accuracy of irradiation treatment is improved. Preparation time and steps for positioning before the treatment are reduced.

In an embodiment, two ends of the main plate in the extension direction have different thicknesses.

In an embodiment, the main plate is detachably connected to a predetermined location on the bearing device by the adapting assembly.

In an embodiment, the main plate is provided with at least one mounting structure for mounting the adapting assembly. The adapting assembly is adjustably mounted in the mounting structure.

In an embodiment, the bearing device includes at least one adapting structure for mounting the adapting assembly. The adapting assembly matches the adapting structure, so as to limit the main plate relative to the bearing device.

In an embodiment, the bearing device includes a treatment loading table. The adapting assembly is arranged between the main plate and the treatment loading table. The adapting assembly includes a first adapting member, a limiting member, and a positioning member. The limiting member is adjustably mounted to the main plate. The first adapting member is adjustably mounted to the main plate. The positioning member is adjustably mounted to the treatment loading table, so as to position the main plate to the treatment loading table.

In an embodiment, the mounting structure includes a first mounting groove. The first adapting member is arranged in a direction perpendicular to the extension direction and adjustably mounted in the first mounting groove.

In an embodiment, the mounting structure further includes a mounting hole. The first adapting member is at least partially accommodated in the first mounting groove and optionally positioned to the main plate by the limiting member. The limiting member protrudes from the first adapting member. The limiting member is optionally assembled in the mounting hole. The mounting hole corresponds to the first mounting groove in location and is used for matching the limiting member.

In an embodiment, the adapting structure includes an adapting groove. The positioning member protrudes from the first adapting member. The positioning member is optionally assembled in the adapting groove.

In an embodiment, the bearing device includes an image loading table. The adapting assembly is arranged between the main plate and the image loading table. The adapting assembly includes a second adapting member. The second adapting member includes an adapting surface. The adapting surface is adjustably matched to a surface and/or an edge of the image loading table.

In an embodiment, the mounting structure includes a second mounting groove. The second adapting member is adjustably mounted in the second mounting groove. The second adapting member is at least partially accommodated in the second mounting groove, so as to limit the main plate relative to the image loading table at least in the direction perpendicular to the extension direction.

In an embodiment, a shape of a loading surface of the treatment loading table is different from that of a loading surface of the image loading table. The loading surface of the treatment loading table is a flat surface. The loading surface of the image loading table is a cambered surface.

A second aspect of the disclosure provides a supporting device. The supporting device includes a main plate used for at least partially bearing an irradiated body. The main plate defines an extension direction, and includes a supporting surface arranged in the extension direction, a side surface connected to the supporting surface, and a back surface arranged opposite the supporting surface. The supporting device further includes at least one diseased part supporting assembly. The diseased part supporting assembly is used for at least partially supporting a diseased part of the irradiated body. The diseased part supporting assembly is detachably connected to the main plate. Preferably, an area of the diseased part supporting assembly is less than that of the main plate. Thus, a special diseased part can be accurately positioned, and unnecessary physical interference can be reduced.

In an embodiment, the diseased part supporting assembly includes a first supporting member at least partially protruding from the side surface and a second supporting member for supporting the first supporting member. The first supporting member is detachably connected to the main plate by the second supporting member. The first supporting member is movable relative to the second supporting member. The second supporting member is movable relative to the main plate. The second supporting member is arranged on the side surface and/or the back surface.

In an embodiment, at least one third mounting groove for accommodating the second supporting member is provided on the side surface.

In an embodiment, the diseased part supporting assembly further includes a locking member arranged on the second supporting member. The locking member includes a driving portion, a pressing portion, and a protrusion. The driving portion is used for driving the locking member to move between a locking location and a releasing location. The pressing portion is used for pressing the locking member in a locking direction. The protrusion is arranged on the driving portion. A locking hole matching the protrusion is provided on the main plate. At the locking location, the locking member is arranged in the third mounting groove, and the protrusion is accommodated in the locking hole, such that the second supporting member is fixed relative to the main plate. At the releasing location, the protrusion is separated from the locking hole, such that the second supporting member is movable relative to the main plate.

In an embodiment, the diseased part supporting assembly further includes a locking member arranged on the second supporting member. The locking member includes a driving portion, a pressing portion, and a protrusion. An included angle delimited by the driving portion, the pressing portion, and the protrusion is not greater than 45°.

In an embodiment, the second supporting member includes a travel limiting member. The travel limiting member is used for limiting displacement of the protrusion of the locking member in at least one direction. Further, the travel limiting member may limit a location of the protrusion in a path in a direction parallel and/or perpendicular to an extension direction of the second supporting member.

In an embodiment, the diseased part supporting assembly further includes a fastening member for connecting the first supporting member to the second supporting member. The fastening member is movably connected to the second supporting member, such that the first supporting member is movable relative to the second supporting member.

In an embodiment, the diseased part supporting assembly further includes an adjusting member. The adjusting member is used for compensating for a gap generated when the first supporting member is positioned.

In an embodiment, the diseased part supporting assembly further includes a shape adapting member and a fixing member. The shape adapting member is used for adapting to a shape of the diseased part of the irradiated body, and includes a hollow structure for a ray to pass through. The fixing member is used for fixing and/or detaching the shape adapting member.

A third aspect of the disclosure provides a method for positioning a supporting device. The method includes: positioning the supporting device to a bearing device, where the bearing device includes a treatment loading table and/or a simulation loading table, and the supporting device includes a main plate and a first adapting member; and mounting the first adapting member to a back surface and/or a side surface of the main plate, where the main plate is connected to a predetermined location on the bearing device by the first adapting member.

According to a method for positioning a supporting device provided in embodiments of the disclosure, a main plate and a diseased part supporting assembly are directly mounted to a treatment loading table in an irradiation treatment room by an adapting assembly such that irradiation treatment can be performed on an irradiated body. When different scenes are required to be switched for the irradiated body or in different treatment phases, through the method, locations of the irradiated body and a diseased part positioned relative to a simulation loading table are recorded in a simulated positioning room. Subsequently, the main plate and the irradiated body can be rapidly and accurately positioned relative to a treatment loading table in an irradiation treatment room according to the locations. Thus, it is ensured that the main plate and the irradiated body are located at same locations as far as possible in different scenes or in different treatment phases. Accuracy of irradiation treatment is improved. Preparation time and steps for positioning before the treatment are reduced.

In an embodiment, the method includes: positioning the main plate to the treatment loading table based on a group of location parameters when the irradiated body is positioned again.

In an embodiment, the bearing device includes an image loading table. The supporting device is positioned to the image loading table. The supporting device further includes a second adapting member. The second adapting member is mounted to the back surface and/or the side surface of the main plate. The main plate is connected to a predetermined location on the image loading table by the second adapting member.

In an embodiment, a second mounting structure is arranged on the back surface and/or the side surface of the main plate. A matching mounting location relative to the image loading table is selected according to a size of the image loading table. The second adapting member is assembled in the second mounting structure. A matching mounting location relative to the image loading table is selected according to a location of the diseased part of the irradiated body. The main plate is mounted.

In an embodiment, an adapting surface is arranged on the second adapting member. The adapting surface is snap-fitted to a surface and/or an edge of the image loading table, so as to limit the main plate relative to the image loading table.

In an embodiment, a shape of a loading surface of the treatment loading table or the simulation loading table is different from that of a loading surface of the image loading table. The method further includes: detaching the supporting device from the bearing device (the treatment loading table or the simulation loading table), detaching the first adapting member from the main plate, and mounting the second adapting member; or detaching the supporting device from the image loading table, detaching the second adapting member from the main plate, and mounting the first adapting member.

In an embodiment, the method further includes: recording location parameters of the main plate and the bearing device when the irradiated body is positioned to the bearing device; and positioning the main plate to the bearing device based on the location parameters before the irradiated body is transferred to the bearing device again.

In an embodiment, the location parameter includes a first location parameter. The method further includes: positioning the supporting device to the bearing device, where at least two first mounting structures are arranged on the back surface and/or the side surface of the main plate; selecting a matching mounting location relative to the first mounting structures according to the location of the diseased part of the irradiated body; assembling the first adapting member in the first mounting structures; and recording the first location parameter.

In an embodiment, the location parameter includes a second location parameter. At least two adapting structures for mounting the first adapting member are arranged on the bearing device. The method further includes: selecting a matching mounting location relative to the adapting structures according to the location of the diseased part of the irradiated body, assembling the main plate in the adapting structures, and recording the second location parameter.

In an embodiment, the method further includes: positioning the diseased part supporting assembly to the main plate based on another group of location parameters when the irradiated body is positioned again.

In an embodiment, the method further includes: positioning a diseased part supporting assembly to the main plate on the bearing device, and recording location parameters of the diseased part supporting assembly and the bearing device when the irradiated body is positioned to the diseased part supporting assembly and the bearing device; and positioning the diseased part supporting assembly to the bearing device based on the location parameters before the irradiated body is transferred to the diseased part supporting assembly and the bearing device again.

In an embodiment, the location parameter includes a third location parameter. The diseased part supporting assembly includes a first supporting member protruding from the side surface of the main plate and a second supporting member. The first supporting member is detachably connected to the main plate by the second supporting member. At least one third mounting structure capable of accommodating the second supporting member is arranged on the main plate. The method further includes: selecting a matching mounting location relative to a beam generating device and the third mounting structure according to the location of the diseased part of the irradiated body, assembling the second supporting member in the third mounting structure, and recording the third location parameter.

In an embodiment, the location parameter includes a fourth location parameter. At least two fourth mounting structures for locking the second supporting member are arranged on the main plate. The method further includes: selecting a matching mounting location relative to the fourth mounting structures according to the location of the diseased part of the irradiated body, assembling the second supporting member in the fourth mounting structures, and recording the fourth location parameter.

In an embodiment, the location parameter includes a fifth location parameter. A fastening member for adjusting and locking the first supporting member is arranged on the second supporting member. The method further includes: selecting a matching mounting location relative to the second supporting member according to the location of the diseased part of the irradiated body, mounting the first supporting member, and recording the fifth location parameter.

A fourth aspect of the disclosure provides a radiotherapy system. The radiotherapy system includes a beam generating device, a bearing device, and a supporting device. The beam generating device is used for generating a treatment beam. The bearing device is used for at least partially bearing an irradiated body. The supporting device is used for at least partially bearing the irradiated body. The supporting device is detachably connected to the bearing device. When the irradiated body is required to be moved to switch to a different bearing device or is required to be treated in different treatment phases, by mounting and positioning the supporting device, it can be still ensured that the irradiated body maintains a predetermined irradiation pose when being positioned again and is not affected by movement and adjustment of the irradiated body. The irradiated body can be further separated from the supporting device during movement, such that no constraint and burden other than treatment exist.

According to the radiotherapy system provided in embodiments of the disclosure, the bearing device includes a treatment loading table and/or an image loading table. In an irradiation positioning room, a main plate is positioned to the treatment loading table by a first adapting member such that irradiation treatment can be performed on the irradiated body. In a medical image room, the main plate is positioned to the image loading table by a second adapting member. Medical image data of the irradiated body is acquired in combination with a medical image device. A location mark is made for a diseased part of the irradiated body. Preferably, a simulated positioning room may be further arranged. In the simulated positioning room, the main plate is positioned to a simulation loading table by the first adapting member. Simulated positioning is performed on the irradiated body according to the medical image data and the location mark of the diseased part in combination with a simulated positioning device, to prepare for the irradiation treatment. Usage of the radiotherapy system is diversified. By switching different adapting assemblies, the main plate and the diseased part supporting assembly can be rapidly and accurately positioned to bearing devices in different scenes or in different treatment phases. Thus, different treatment requirements of the irradiated body in different scenes or in different treatment phases can be satisfied.

In an embodiment, the supporting device includes a main plate and an adapting assembly. The main plate is detachably connected to the bearing device by the adapting assembly. Two ends of the main plate in an extension direction have different thicknesses.

In an embodiment, the bearing device includes a treatment loading table and an image loading table. The treatment loading table is used for at least bearing the irradiated body during irradiation treatment. The image loading table is used for at least bearing the irradiated body during image acquisition. The supporting device includes the main plate and the adapting assembly. The main plate is detachably connected to the treatment loading table or the image loading table by the adapting assembly.

In an embodiment, the radiotherapy system further includes an irradiation positioning device. The irradiation positioning device is used for performing irradiation positioning on the irradiated body. The treatment loading table is arranged in the irradiation positioning device and used for at least bearing the irradiated body during the irradiation positioning on the irradiated body.

In an embodiment, the radiotherapy system further includes a medical image device. The medical image device is used for acquiring medical image data of the irradiated body. The image loading table is arranged in the medical image device and used for at least bearing the irradiated body when the medical image data of the irradiated body is acquired.

In an embodiment, the bearing device further includes a simulation loading table. The simulation loading table is used for at least bearing the irradiated body during non-irradiation treatment. The main plate is detachably connected to the simulation loading table by the adapting assembly.

In an embodiment, the radiotherapy system further includes a simulated positioning device. The simulated positioning device is used for performing simulated positioning on the irradiated body. The simulation loading table is arranged in the simulated positioning device and used for at least bearing the irradiated body during the simulated positioning on the irradiated body.

In an embodiment, a shape of the treatment loading table is different from that of the image loading table. Further, a shape of a loading surface of the treatment loading table is different from that of a loading surface of the image loading table. The shape of the loading surface of the treatment loading table is the same as that of a loading surface of the simulation loading table.

In an embodiment, the adapting assembly is arranged between the main plate and the bearing device. The adapting assembly includes a first adapting member, a limiting member, and a positioning member. The first adapting member is adjustably mounted to the main plate. The limiting member is adjustably mounted to the main plate. The positioning member is adjustably mounted to the bearing device, such that the main plate is positioned to the bearing device.

In an embodiment, a first mounting groove and a mounting hole corresponding to the first mounting groove in location are provided on the main plate. The first adapting member is arranged in a direction perpendicular to the extension direction and optionally assembled in the first mounting groove. The limiting member protrudes from the first adapting member and is optionally assembled in the mounting hole.

In an embodiment, an adapting groove is provided on the bearing device. The positioning member protrudes from the first adapting member and is optionally assembled in the adapting groove.

In an embodiment, the adapting assembly is arranged between the main plate and the image loading table. The adapting assembly includes a second adapting member. The second adapting member includes an adapting surface. The adapting surface is adjustably matched to a surface and/or an edge of the image loading table.

In an embodiment, a second mounting groove is provided on the main plate. The second adapting member is at least partially accommodated in the second mounting groove, so as to limit the main plate relative to the image loading table at least in the direction perpendicular to the extension direction.

In an embodiment, the radiotherapy system further includes at least one diseased part supporting assembly. The diseased part supporting assembly is used for at least partially supporting the diseased part of the irradiated body. The diseased part supporting assembly is detachably connected to the bearing device or the main plate thereof. Preferably, an area of the diseased part supporting assembly is less than that of the bearing device or the main plate thereof. Thus, a special diseased part can be accurately positioned, and unnecessary physical interference can be reduced.

In an embodiment, the bearing device or the main plate thereof includes a supporting surface, a side surface connected to the supporting surface, and a back surface arranged opposite the supporting surface. The diseased part supporting assembly includes a first supporting member at least partially protruding from the side surface and a second supporting member. The first supporting member is detachably connected to the bearing device or the main plate thereof by the second supporting member. The first supporting member is movable relative to the second supporting member. The second supporting member is movable relative to the bearing device or the main plate thereof. The second supporting member is arranged on the side surface and/or the back surface.

In an embodiment, at least one third mounting groove for accommodating the second supporting member is provided on the side surface.

In an embodiment, the diseased part supporting assembly further includes a locking member arranged on the second supporting member. The locking member includes a driving portion, a pressing portion, and a protrusion. The driving portion is used for driving the locking member to move between a locking location and a releasing location. The pressing portion is used for pressing the locking member in a locking direction. The protrusion is arranged on the driving portion. A locking hole matching the protrusion is provided on the bearing device or the main plate. At the locking location, the locking member is arranged in the third mounting groove, and the protrusion is accommodated in the locking hole, such that the second supporting member is fixed relative to the bearing device or the main plate. At the releasing location, the protrusion is separated from the locking hole, such that the second supporting member is movable relative to the bearing device or the main plate.

In an embodiment, the diseased part supporting assembly further includes a locking member arranged on the second supporting member. The locking member includes a driving portion, a pressing portion, and a protrusion. An included angle delimited by the driving portion, the pressing portion, and the protrusion is not greater than 45°.

In an embodiment, the second supporting member includes a travel limiting member. The travel limiting member is used for limiting displacement of the protrusion of the locking member in at least one direction. Further, the travel limiting member may limit a location of the protrusion in a path in a direction parallel and/or perpendicular to an extension direction of the second supporting member.

In an embodiment, the diseased part supporting assembly further includes a fastening member for connecting the first supporting member to the second supporting member. The fastening member is movably connected to the second supporting member, such that the first supporting member is movable relative to the second supporting member.

In an embodiment, the diseased part supporting assembly further includes an adjusting member. The adjusting member is used for compensating for a gap generated when the first supporting member is positioned.

In an embodiment, the diseased part supporting assembly further includes a shape adapting member and a fixing member. The shape adapting member is used for adapting to a shape of the diseased part of the irradiated body, and includes a hollow structure for a ray to pass through. The fixing member is used for fixing and/or detaching the shape adapting member.

According to a supporting device, a method for positioning a supporting device, and a radiotherapy system in the disclosure, positioning postures of an irradiated body in different scenes and in different phases can be achieved. Unnecessary constraints and limitations on the irradiated body are reduced. Mounting, positioning, and supporting can be rapidly and simply performed. Moreover, accuracy of inspection or treatment is further improved.

It should be understood that within the scope of the disclosure, the above technical features of the disclosure and technical features specifically described in the following text (such as embodiments) can be combined with one another to form new or preferred technical solutions.

### BRIEF DESCRIPTION OF THE DRAWINGS

FIG. 1 is a schematic structural diagram of a supporting surface and a side surface according to an embodiment of the disclosure;
FIG. 2 is a schematic structural diagram of a back surface of a main plate according to an embodiment of the disclosure;
FIG. 3 is a schematic diagram showing that a main plate is positioned to a treatment loading table or a simulation loading table according to an embodiment of the disclosure;
FIG. 4 is an exploded view of a structure in which a main plate is positioned to a treatment loading table or a simulation loading table by a first adapting member according to an embodiment of the disclosure;
FIG. 5 is an exploded view of a structure in which a main plate is positioned to an image loading table by a second adapting member according to an embodiment of the disclosure;
FIG. 6 is a schematic diagram showing that a diseased part supporting assembly is mounted to a front surface of a bearing device according to an embodiment of the disclosure;
FIG. 7 is a schematic locational diagram showing that a diseased part supporting assembly is mounted to a back surface of a bearing device according to an embodiment of the disclosure;
FIG. 8 is a schematic diagram of an entire structure of a diseased part supporting assembly according to an embodiment of the disclosure;
FIG. 9 is a schematic diagram of an entire structure of a diseased part supporting assembly according to another embodiment of the disclosure;
FIG. 10 is a schematic diagram of an entire structure of a diseased part supporting assembly according to yet another embodiment of the disclosure;
FIG. 11 is an enlarged view of a structure of portion M in FIG. 9 according to another embodiment of the disclosure;
FIG. 12 is a schematic diagram showing that a diseased part supporting assembly is mounted to a bearing device according to another embodiment of the disclosure;
FIG. 13 is a schematic diagram showing that a shape adapting member is wrapped around a diseased part of an irradiated body according to an embodiment of the disclosure; and
FIG. 14 is a schematic diagram showing that an irradiated body is subjected to irradiation treatment in an irradiation treatment room according to an embodiment of the disclosure.

Reference numerals in figures: main plate 100, supporting surface 100a, side surface 100b, back surface 100c, first mounting groove 101a, mounting hole 101b, second mounting groove 102, third mounting groove 103, locking hole 104, treatment loading table 201, adapting groove 201a, image loading table 202, simulation loading table 203, first adapting member 301, limiting member 302, positioning member 303, second adapting member 401, adapting surface 401a, limiting surface 401b, diseased part supporting assembly 500, 500', first supporting member 501, 501', second supporting member 502, 502', locking member 503, 503', driving portion 504, 504', pressing portion 505, 505', protrusion 506, 506', fastening member 507, 507', through hole 508, 508', adjusting member 509, mounting plate 510, shape adapting member 511, assembly hole 512, snap-fitting member 513, travel limiting member 514, beam generating device 601, beam shaping body 602, collimator 603, and irradiation treatment room 604.

### DETAILED DESCRIPTION

To make the above objectives, features and advantages of the disclosure more apparent and understandable, particular implementations of the disclosure will be described in detail below in combination with accompanying drawings. Plenty of specific details are set forth in the following descriptions to facilitate full understanding of the disclosure. However, the disclosure can be implemented in many other ways different from those described herein. A person skilled in the art can make similar improvements without departing from the connotation of the disclosure. Thus, the disclosure is not limited by the particular embodiments disclosed below.

In the description of the disclosure, it should be understood that the terms "central", "longitudinal", "transverse", "length", "width", "thickness", "upper", "lower", "front", "back", "left", "right", "vertical", "horizontal", "top", "bottom", "inner", "outer", "clockwise", " counterclockwise", "axial", "radial", "circumferential", etc. indicate orientation or location relationships based on those shown in the accompanying drawings, are merely for ease of description of the disclosure and simplicity of description, and are not intended to indicate or imply that a referred device or element must have a particular orientation and be constructed and operated in a particular orientation, and thus cannot be construed as a limitation to the disclosure.

In addition, terms "first" and "second" are used merely for the purpose of description, and should not be construed as indicating or implying relative importance or implying a number of indicated technical features. Thus, a feature defined by "first" or "second" may explicitly indicate or implicitly include at least one of such features. In the description of the disclosure, "plurality of" means at least two, for example, two and three, unless otherwise explicitly and specifically defined.

In the disclosure, unless otherwise explicitly specified and defined, terms "mounted to", "connected to", "connection", "fixed to", etc. should be understood in a broad sense. For example, they may denote a fixed connection, a detachable connection, or an integrated connection, denote a mechanical connection or an electrical connection, denote a direct connection, or an indirect connection via an intermediate medium, or denote communication of interiors of two elements or an interaction between two elements, unless otherwise explicitly defined. A person of ordinary skill in the art can understand the specific meanings of the above terms in the disclosure according to specific situations.

In the disclosure, unless otherwise explicitly specified and defined, a case that a first feature is "above" or "below" a second feature may denote that the first feature is in direct contact with the second feature, or the first feature is in indirect contact with the second feature by an intermediary. Moreover, a case that the first feature is "above", "over", or "on" the second feature may denote that the first feature is exactly or not exactly above the second feature, or merely denote that a level of the first feature is higher than that of the second feature. A case that the first feature is "below", "under", and "beneath" the second feature may denote that the first feature is exactly or not exactly below the second feature, or merely denote that a level of the first feature is lower than that of the second feature.

It should be noted that in a case that an element is referred to as being "fixed to" or "arranged on" another element, the element may be directly on another element or an intermediate element may exist. In a case that an element is considered as being in "connected" to another element, the element may be directly connected to another element or an intermediate element may exist at the same time. Terms "perpendicular", "horizontal", "upper", "lower", "left", "right", and similar expressions used herein are used for illustrative purposes only and do not denote unique implementations.

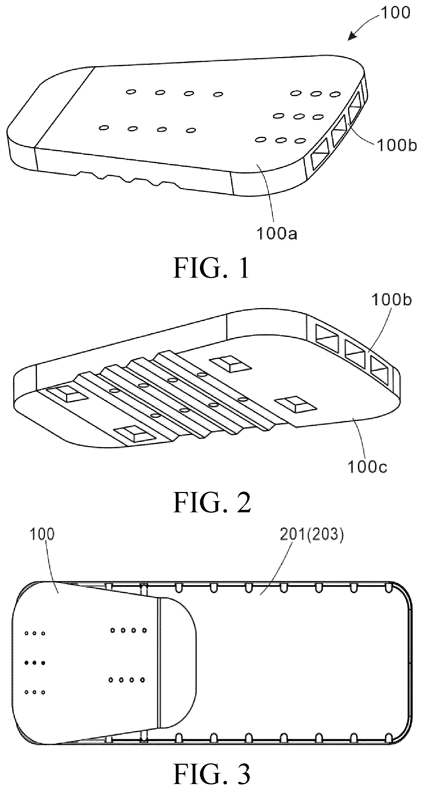

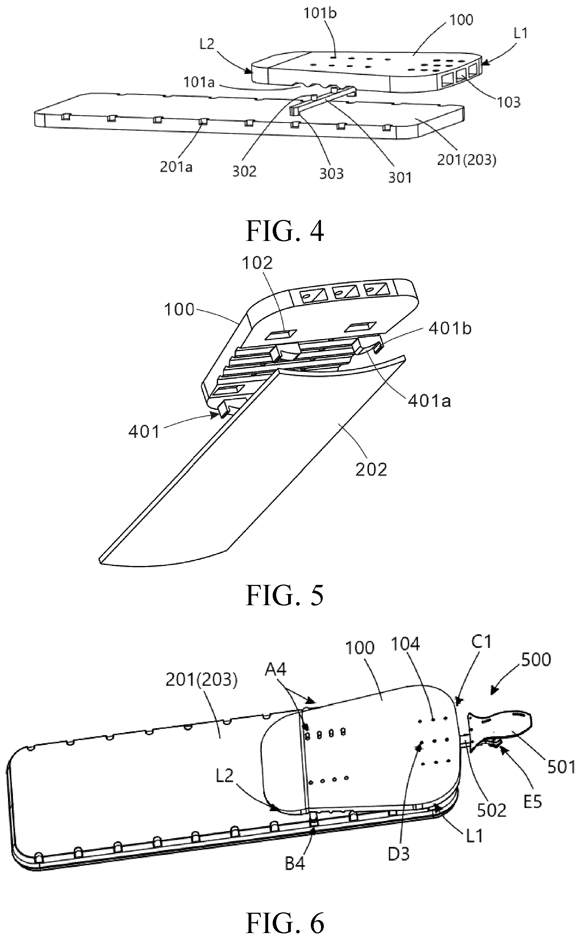

With reference to FIG. 1, a schematic structural diagram of a supporting surface and a side surface of a main plate of a supporting device according to an embodiment of the disclosure is shown in FIG. 1. A supporting device includes a main plate 100 and an adapting assembly. The main plate 100 is used for at least partially bearing an irradiated body. The main plate 100 defines an extension direction. The main plate 100 includes a supporting surface 100a arranged in the extension direction, a side surface 100b connected to the supporting surface 100a, and a back surface 100c arranged opposite the supporting surface 100a. The main plate 100 is set as plate/block/platform-shaped. The main plate 100 is used for partially supporting the irradiated body. Specifically, the main plate 100 can support a back and/or a shoulder of the irradiated body. The main plate 100 is further used for being loaded onto a bearing device for bearing the irradiated body. Specifically, the bearing device may include different shapes, such as a shape (with reference to FIG. 3 and FIG. 4) of a treatment loading table 201 and a shape (with reference to FIG. 5) of an image loading table 202. The main plate 100 is loaded onto the bearing device such that the irradiated body can be positioned and a diseased part supporting assembly 500 (with reference to FIG. 6 to FIG. 13) can be mounted.

With reference to FIG. 2, a schematic structural diagram of a back surface of a main plate of a supporting device according to an embodiment of the disclosure is shown in FIG. 2. An adapting assembly is arranged on a back surface 100c and/or a side surface 100b of a main plate 100 and used for connecting the main plate 100 to a predetermined location. The main plate 100 is detachably connected to the predetermined location on a bearing device by the adapting assembly. During irradiation treatment, an irradiated body may be generally required to be moved to bearing devices in different scenes, or irradiation preparation or treatment is not performed in a same time period. Since the main plate 100 can be detachably mounted and positioned to different bearing devices by the adapting assembly, the main plate 100 and/or at least one diseased part supporting assembly 500 (with reference to FIG. 6 to FIG. 13) can be positioned to the bearing devices. Irradiated bodies having different body shapes and diseased parts thereof can be suitably supported and positioned such that subsequent irradiation treatment can be facilitated. It can be easily understood that the back surface or the side surface of the main plate may have a boundary line or may have no clear boundary line. In some embodiments, the supporting surface of the main plate may smoothly transition to the back surface of the main plate. When the irradiated body is required to be moved to switch to a different scene or is required to be supported and positioned in different phases (for example, interval time is greater than or equal to 12 hours), a worker is only required to mount and position the main plate and/or the diseased part supporting assembly of the supporting device. Through adjustment and fixation, it can be still ensured that after an interval time period, the irradiated body maintains a predetermined pose when being positioned again and is not affected by movement and adjustment of the irradiated body in the interval time period. Further, the irradiated body can be separated from the supporting device during movement in different phases or in a same phase. That is, the movement of the irradiated body cannot be limited by the main plate. The irradiated body itself can be transferred to different scenes. Moreover, discomfort caused by an additional constraint and burden for bearing the main plate, etc. cannot occur.

In some embodiments, the main plate 100 is provided with at least one mounting structure for mounting an adapting assembly. The adapting assembly is adjustably mounted in the mounting structure. Preferably, at least two mounting structures are included such that the adapting assembly can match the mounting structures at different locations. Thus, the adapting assembly can be adjustably mounted to the main plate 100.

In some embodiments, the bearing device includes at least one adapting structure for mounting the adapting assembly. The adapting assembly matches the adapting structure, such that the main plate 100 is limited relative to a loading table (the bearing device). Preferably, at least two adapting structures are included such that the adapting assembly can match the adapting structures at different locations. Thus, the main plate 100 can include at least two locations relative to the loading table.

With reference to FIG. 3 and FIG. 4, an exploded view of a structure in which a main plate of a supporting device is positioned to a treatment loading table or a simulation loading table by a first adapting member according to an embodiment of the disclosure is shown in FIG. 4. A loading table includes a treatment loading table 201. An adapting assembly is arranged between the main plate 100 and the treatment loading table 201. The adapting assembly includes a first adapting member 301, a limiting member 302, and a positioning member 303. The limiting member 302 is adjustably mounted to the main plate 100. The first adapting member 301 is adjustably mounted to the main plate 100. The positioning member 303 is adjustably mounted to the treatment loading table 201, such that the main plate 100 is positioned to the treatment loading table 201. The treatment loading table 201 is used for bearing an irradiated body. The treatment loading table 201 may be a device for bearing the irradiated body, such as a treatment bed. The main plate 100 is located between the irradiated body and the treatment loading table 201. The main plate 100 is movable relative to the treatment loading table 201 such that the main plate 100 can be adjusted to a location for suitably supporting the irradiated body, to match irradiated bodies having different body shapes. The main plate 100 is positioned by the first adapting member 301, the limiting member 302, and the positioning member 303, such that the main plate 100 is fixed relative to the treatment loading table 201.

In some embodiments, the mounting structure includes a first mounting groove 101a. The first adapting member 301 is arranged in a direction perpendicular to an extension direction and adjustably mounted in the first mounting groove 101a. The first adapting member 301 is set as a rod-shaped structure having a length. Preferably, a length of the first adapting member 301 is equal to a width of the treatment loading table 201. In the embodiment, the first adapting member 301 is a long strip-shaped rod, and is adjustably mounted in the first mounting groove 101a. A matching groove-shaped structure of the first mounting groove 101a is selected according to a location of a diseased part of the irradiated body and a preliminarily-positioned irradiation location. The first adapting member 301 is assembled, such that the first adapting member 301 is positioned to the main plate 100.

In some embodiments, the mounting structure further includes a mounting hole 101b. The first adapting member 301 is at least partially accommodated in the first mounting groove 101a and optionally positioned to the main plate 100 by the limiting member 302. The limiting member 302 protrudes from the first adapting member 301. The limiting member 302 is optionally assembled in the mounting hole 101b. The mounting hole 101b corresponds to the first mounting groove 101a in location and is used for matching the limiting member 302. The limiting member 302 may include a plurality of protrusions 506. The plurality of protrusions 506 are arranged on the first adapting member 301 at discrete intervals. Preferably, the limiting member 302 and the first adapting member 301 are integrally formed. The limiting member 302 is adjustably mounted in the mounting hole 101b, such that the limiting member 302 is positioned to the main plate 100.

In some embodiments, the adapting structure includes an adapting groove 201a. The positioning member 303 protrudes from the first adapting member 301. The positioning member 303 is optionally assembled in the adapting groove 201a. The positioning member 303 may include two positioning blocks arranged at two ends of the first adapting member 301 respectively. Preferably, the positioning member 303 and the first adapting member 301 are integrally formed. The positioning member 303 is optionally assembled in the adapting groove 201a, such that the positioning member 303 is positioned to the treatment loading table 201. After relative locations of the main plate 100 and the treatment loading table 201 are determined, the main plate 100 is fixed to the treatment loading table 201 by the first adapting member 301, the limiting member 302, and the positioning member 303, such that the main plate 100 is fixed relative to the treatment loading table 201. When the irradiated body is located to the main plate 100, the irradiated body is positioned relative to a predetermined location on the treatment loading table 201.

Preferably, the loading table may further include a simulation loading table 203. The simulation loading table 203 has a width the same as that of the treatment loading table 201 and includes at least a similar adapting structure.

In some embodiments, the first mounting groove 101a may include elongated slots provided on the back surface 100c of the main plate 100. The elongated slots are arranged in an array. The slot is used for accommodating the first adapting member 301 such that the main plate 100 can be steadily limited to the treatment loading table 201. The mounting hole 101b corresponds to the first mounting groove 101a in location and is used for matching the limiting member 302 protruding from the first adapting member 301 accommodated in the first mounting groove 101a. That is, by embedding the first adapting member 301 in the first mounting groove 101a and embedding the limiting member 302 in the mounting hole 101b, the main plate 100 is fixed relative to the first adapting member 301.

In some embodiments, the adapting groove 201a may include at least two slots provided in pairs. The two slots are preferably set as U-shaped. Preferably, U-shaped openings of the two slots are provided in opposite directions. The two slots are provided at an edge of a surface of the treatment loading table 201. The positioning member 303 can be embedded in the two slots. That is, by embedding the positioning member 303 in the two slots provided in pairs on the treatment loading table 201, the first adapting member 301 is fixed relative to the treatment loading table 201. In addition, a shape of the adapting groove 201a is not limited. The adapting groove may be a closed slot, or an elongated slot in a direction perpendicular to an extension direction on the surface of the treatment loading table 201, as long as the adapting groove 201a can limit a location of the first adapting member 301.

In some embodiments, the main plate 100 is adjustable relative to the treatment loading table 201 by increasing a number of adapting grooves 201a on the treatment loading table 201 and numbers of first mounting grooves 101a and mounting holes 101b on the main plate 100. Preferably, in a case that a plurality of adapting grooves 201a are provided, the plurality of adapting grooves 201a are preferably provided on the treatment loading table 201 in the extension direction. After a location of the main plate 100 relative to the treatment loading table 201 is selected, the positioning member 303 can be embedded in any suitable adapting groove 201a to position the main plate 100. A plurality of first mounting grooves 101a are provided. The plurality of first mounting grooves 101a are provided on the main plate 100 in the extension direction. After the location of the main plate 100 relative to the treatment loading table 201 is selected, the first adapting member 301 can be embedded in any suitable first mounting groove 101a to position the main plate 100 such that the main plate can be limited relative to the treatment loading table in the extension direction of the main plate. A plurality of mounting holes 101b are provided. The plurality of mounting holes 101b are provided on the main plate 100 in the extension direction. After the location of the main plate 100 relative to the treatment loading table 201 is selected, the limiting member 302 can be embedded in any suitable mounting hole 101b to position the main plate 100 such that the main plate can be limited relative to the treatment loading table in a direction perpendicular to the extension direction. A plurality of adapting grooves 201a may be provided only on the treatment loading table 201, or a plurality of first mounting grooves 101a and a plurality of mounting holes 101b may be provided only on the main plate 100. Preferably, two or more adapting grooves 201a, two or more first mounting grooves 101a, and two or more mounting holes 101b are provided. Thus, the main plate 100 can be more reliably moved and positioned relative to the treatment loading table 201.

In some embodiments, the above carrier structure, that is, the treatment loading table 201 may be replaced with the simulation loading table 203.

With reference to FIG. 5, an exploded view of a structure in which a main plate of a supporting device is positioned to an image loading table by a second adapting member according to an embodiment of the disclosure is shown in FIG. 5. A loading table includes an image loading table 202. An adapting assembly is arranged between a main plate 100 and the image loading table 202. The adapting assembly includes a second adapting member 401. The second adapting member 401 includes an adapting surface 401a. The adapting surface 401a is adjustably matched to a surface and/or an edge of the image loading table 202. The image loading table 202 may be a bearing device for bearing an irradiated body for image scanning. When the image loading table 202 is arranged in a medical image irradiation room for the image scanning, the main plate 100 and the image loading table 202 jointly support the irradiated body. After the main plate 100 is adjusted to a predetermined location relative to the image loading table 202, a location of the main plate 100 is positioned relative to the image loading table 202 by the second adapting member 401. The adapting surface 401a is used for at least partially abutting against a surface and/or an edge of the image loading table 202, such that the main plate 100 is positioned to the image loading table 202.

In some embodiments, the second adapting member 401 is optional and replaceable. According to image mounting tables 202 of different models, surfaces of the image mounting tables 202 are also different. By replacing a corresponding second adapting member 401, an adapting surface 401a of the second adapting member can match the surface and/or the edge of the image loading table 202. Thus, the main plate 100 can be positioned to the image loading table 202 by the second adapting member 401.

In some embodiments, during computed tomography (CT), an image loading table 202 for scanning has a cambered section. That is, the image loading table 202 has a curved surface. In this case, the second adapting member 401 may further include an adapting surface 401a and a limiting surface 401b. The adapting surface 401a is a surface matching the surface of the image loading table 202. The second adapting member 401 is in snap fit with the image loading table 202 by attaching the adapting surface 401a to a surface of the edge of the image loading table 202 and attaching the limiting surface 401b to a side surface of the image loading table 202. Thus, the main plate 100 can be reliably positioned to the image loading table 202. Moreover, the limiting surface 401b limits the main plate 100 relative to the image loading table 202 in at least a direction perpendicular to an extension direction.

In some embodiments, during magnetic resonance imaging (MRI), an image loading table 202 for scanning is straight-plate shaped (not shown in the figure). That is, the image loading table 202 has a flat surface. In this case, the second adapting member is replaced with a second adapting member 401 of which an adapting surface 401a is a flat surface. A slot matching the second adapting member 401 is provided on the surface of the image loading table 202 correspondingly such that the second adapting member 401 can be embedded in the slot of the image loading table 202. Thus, the second adapting member 401 can be in snap fit with the image loading table 202.

In some embodiments, the two types of second adapting members 401 are detachable relative to the main plate 100 and can be alternatively used according to image loading tables 202 having different shapes and different surfaces. During positioning, each type of second adapting members 401 preferably appear in pairs. For example, two or four second adapting members are arranged. Thus, the main plate 100 and the irradiated body can be further limited in the direction perpendicular to the extension direction, and the irradiated body can be prevented from unnecessarily sliding on the loading table. When the irradiated body is required to be moved to switch to a different loading table or is required to be positioned to a same loading table again according to a requirement, in some embodiments, the irradiated body may be entirely transferred without positioning adjustment. In some other embodiments, a worker may be only required to mount and position the main plate and/or a diseased part supporting assembly of the supporting device. Through adjustment and fixation, it can be still ensured that subsequently the irradiated body maintains a predetermined positioned pose and is not affected by movement and adjustment of the irradiated body. Moreover, no additional constraint and burden exist during the movement of the irradiated body.

In some embodiments, the mounting structure includes a second mounting groove 102. The second adapting member 401 is adjustably mounted in the second mounting groove 102. The second adapting member 401 is at least partially accommodated in the second mounting groove 102, such that the main plate 100 is limited relative to the image loading table 202 at least in the direction perpendicular to the extension direction. The second mounting groove 102 is provided on a back surface 100c and/or a side surface 100b of the main plate 100 and is used for mounting the second adapting member 401. The second adapting member 401 is detachably and adjustably mounted in the second mounting groove 102 such that a location of the second adapting member can be adjusted or the second adapting member can be replaced with a different second adapting member 401. Preferably, different mounting locations may be selected in the second mounting groove 102 in the direction perpendicular to the extension direction, to match loading tables having different widths. Since the second adapting member 401 is detachably mounted in the second mounting groove 102, different second adapting members 401 are mounted according to different image mounting tables 202. For example, if the second adapting member 401 is still not applicable to a selected image loading table 202 after being adjusted in the second mounting groove 102, it is considered that the second adapting member is replaced with a suitable second adapting member 401. When the second adapting member 401 is not required, a worker can simply take out the second adapting member 401 from the second mounting groove 102, to avoid an impact on another applicable scene.

With reference to FIG. 6, a schematic diagram showing that a diseased part supporting assembly of a supporting device is mounted to a main plate on a bearing device according to an embodiment of the disclosure is shown in FIG. 6. A diseased part supporting assembly 500 is used for at least partially supporting a diseased part of an irradiated body. The diseased part supporting assembly 500 is detachably connected to a main plate 100. The diseased part supporting assembly 500 is used for supporting and positioning the diseased part of the irradiated body. The diseased part supporting assembly 500 is mounted by the main plate 100. That is, the diseased part of the irradiated body is supported by the diseased part supporting assembly 500 connected to the main plate 100. The diseased part supporting assembly 500 is movable relative to the main plate 100, and is used for adjusting a location of the diseased part, such that the diseased part is subjected to irradiation treatment at a more suitable location. The diseased part supporting assembly 500 is arranged on the main plate 100. The diseased part supporting assembly 500 is fixed and mounted by the main plate 100. Moreover, the main plate 100 is positioned to a treatment loading table 201 and/or an image loading table 202 by an adapting assembly. Thus, a problem that the diseased part supporting assembly 500 is complexly mounted in the past is effectively resolved. The diseased part of the irradiated body is supported. Specifically, a head, a hand, or a leg, etc. of the irradiated body is supported. The diseased part supporting assembly 500 is movable relative to the main plate 100. By adjusting a location of the diseased part supporting assembly, a location of the diseased part of the irradiated body is adjusted in a targeted manner. Thus, the diseased part is adjusted to a suitable treatment location.

In some embodiments, the bearing device includes a supporting surface, a side surface connected to the supporting surface, and a back surface arranged opposite the supporting surface. Thus, the above diseased part supporting assembly may be directly mounted to the bearing device.

In some embodiments, in combination with FIG. 4 and FIG. 6, in an extension direction of the main plate 100, one end for accommodating the diseased part supporting assembly has a thickness of L1, and the other end linked to a surface of the bearing device has a thickness of L2. Preferably, two ends of the main plate 100 in the extension direction has different thicknesses. A supporting surface of the main plate 100 has slope. That is, L1 is greater than L2. Further, in this way, the main plate 100 can smoothly transition to the surface of the bearing device. By such a transition surface, mounting of the diseased part supporting assembly is taken into account while positioning comfort and stability of the irradiated body are improved.

In some embodiments, an area of the diseased part supporting assembly 500 is less than an area of the main plate 100 or a loading table. That is, the diseased part supporting assembly 500 having a less area protrudes from the main plate 100 and/or the loading table having a greater area. Thus, interference of a body of the supporting device can be reduced, and the diseased part can be closer to a beam exit of a collimator. In this way, a beam can accurately irradiate some special diseased parts, to ensure an effective treatment effect.

In some embodiments, the diseased part supporting assembly 500 includes a first supporting member 501 at least partially protruding from a side surface 100b and a second supporting member 502. The first supporting member 501 is detachably connected to the main plate 100 by the second supporting member 502. The first supporting member 501 is movable relative to the second supporting member 502. The second supporting member 502 is movable relative to the main plate 100. The second supporting member 502 is arranged on the side surface 100b and/or the back surface 100c. The first supporting member 501 is used for supporting the diseased part of the irradiated body. The second supporting member 502 is used for connecting the main plate 100 and the first supporting member 501, and plays a role of the first supporting member 501. Specifically, the first supporting member 501 may be a head stay, a head rest, a hand stay, a hand rest, a leg rest, a foot rest, etc. The second supporting member 502 may be rod/column/block-shaped. In the embodiment, the second supporting member 502 is set as a rectangular column body having a length. One end of the second supporting member 502 is adjustably and fixedly connected to the main plate 100. The other end of the second supporting member is connected to a bottom of the first supporting member 501.

In some embodiments, adjustment on the diseased part of the irradiated body includes angle adjustment, front-back adjustment, and left-right adjustment. The angle adjustment is implemented by rotating the first supporting member 501 relative to the second supporting member 502. The left-right adjustment is implemented by arranging the second supporting member 502 in third mounting grooves 103 provided at different locations on the main plate 100. The front-back adjustment is implemented by selecting a depth of a slide adjustment for mounting the second supporting member 502 in a selected third mounting groove 103. After the adjustment, the diseased part keeps a suitable treatment distance from the exit of the collimator. Thus, a treatment solution is optimized, and subsequent irradiation treatment by the beam on the diseased part is facilitated.

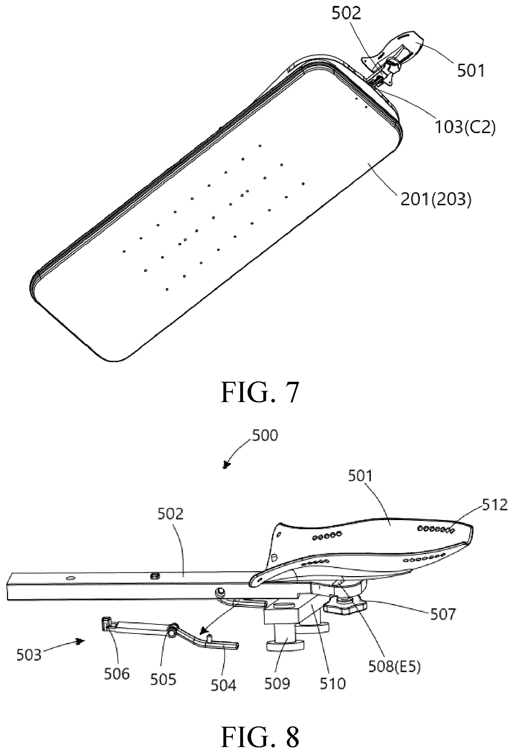

With reference to FIG. 7, a schematic locational diagram showing that a diseased part supporting assembly of a supporting device is mounted to a third mounting groove on a main plate on a loading table according to an embodiment of the disclosure is shown in FIG. 7. At least one third mounting groove 103 for accommodating a second supporting member 502 is provided on a side surface 100b, such that the second supporting member 502 includes at least one mounting location relative to the main plate 100. In combination with FIG. 4, the third mounting groove 103 is used for mounting the second supporting member 502. The third mounting groove 103 is further used for adjusting a location of the second supporting member 502 relative to the main plate 100. The third mounting groove 103 has a depth. The depth is set in an extension direction. A portion of the second supporting member 502 can move back and forth in the third mounting groove 103. That is, front-back adjustment on the second supporting member 502 can be implemented. After movement and mounting, a first supporting member 501 can be aligned with a location of a diseased part to support the diseased part. Preferably, at least two third mounting groove 103 for accommodating the second supporting member 502 are provided on the side surface 100b, such that the second supporting member 502 includes at least two mounting locations relative to the main plate 100. Preferably, two or more third mounting grooves 103 are provided in a direction perpendicular to the extension direction at a discrete interval/discrete intervals. The second supporting member 502 can be mounted to any third mounting groove 103 according to a requirement. That is, the second supporting member 502 can be moved left and right and mounted. In cases of locations of different diseased parts, a location of one third mounting groove 103 can make the first supporting member 501 closer to an exit of a collimator. Thus, it can be ensured that the locations of the diseased parts can be accurately irradiated.

With reference to FIG. 8, a schematic diagram of an entire structure of a diseased part supporting assembly of a supporting device according to an embodiment of the disclosure is shown in FIG. 8. The diseased part supporting assembly 500 further includes a locking member 503 arranged on a second supporting member 502. The locking member 503 includes a driving portion 504, a pressing portion 505, and a protrusion 506. The driving portion 504 drives the locking member 503 to move between a locking location and a releasing location. The pressing portion 505 is used for pressing the locking member 503 in a locking direction. The protrusion 506 is arranged on an opposite side of the driving portion 504. The locking direction is a direction in which the locking member 503 is locked. In combination with FIG. 6, a locking hole 104 matching the protrusion 506 is provided on the main plate 100. In the locking location, the locking member 503 is arranged in the third mounting groove 103, and the protrusion 506 is accommodated in the locking hole 104, such that the second supporting member 502 is fixed relative to the main plate 100. In the releasing location, the protrusion 506 is separated from the locking hole 104, such that the second supporting member 502 is movable relative to the main plate 100. The locking member 503 may be at least partially accommodated in the third mounting groove 103 together with the second supporting member 502. After left-right adjustment and front-back adjustment on the second supporting member 502 are completed in the third mounting groove 103, the locking member 503 fixes and locks the diseased part supporting assembly 500. The driving portion 504 is used for adjusting the locations of the locking member 503, that is, adjusting the locking member from the locking location to the releasing location or from the releasing location to the locking location. The pressing portion 505 is used for ensuring that the locking member 503 is always located at the locking location in a non-operating state. The protrusion 506 is used for locking the second supporting member 502 in combination with the locking hole 104.

In some embodiments, preferably, the driving portion 504 is preferably rod-shaped. A recess (not shown in the figure) is provided on a lower side of the second supporting member 502. The driving portion 504 is at least partially accommodated in the recess. With reference to FIG. 8, the driving portion 504 and the pressing portion 505 at one end of the locking member 503 and the protrusion 506 at the other end of the locking member 503 are not located in one plane. Preferably, an included angle delimited by the driving portion, the pressing portion, and the protrusion is not greater than 45°. Preferably, one end of the driving portion 504 is arranged in the recess and movably connected to the second supporting member 502. The pressing portion 505 is a distortion spring including a pivot axis, and is arranged at a joint between one end of the driving portion 504 and the second supporting member 502. The pressing portion 505 applies a press force to the driving portion 504. In the non-operating state, the other end of the driving portion 504 is moved away from the second supporting member 502 correspondingly. At this time, the press force generated by the pressing portion 505 makes the protrusion 506 accommodated in the locking hole 104, to ensure that the locking member 503 is located at the locking location. Thus, the second supporting member 502 is fixed relative to the main plate 100. In an operating state, when an external force is applied to the driving portion 504, the other end of the driving portion 504 is pressed close to the second supporting member 502. Moreover, the protrusion 506 originally accommodated in the locking hole 104 is moved away from the second supporting member 502. At this time, the protrusion 506 is separated from the locking hole 104, and the locking member 503 is located at the releasing location. Thus, the second supporting member 502 can be moved in the third mounting groove 103 for adjustment.

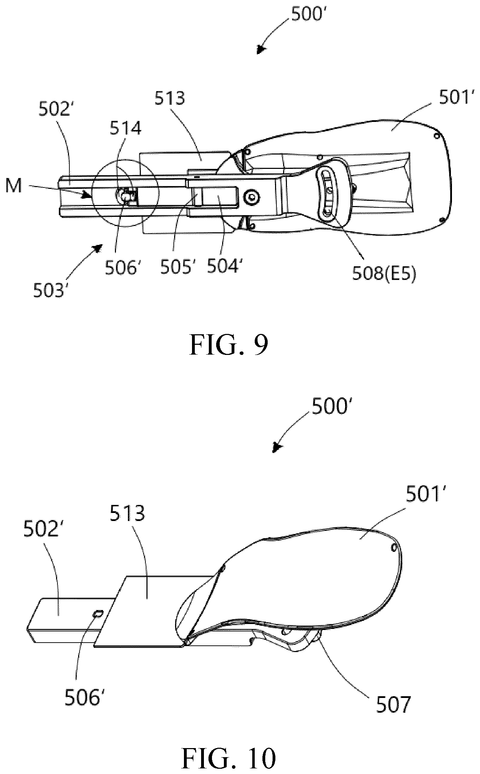

With reference to FIG. 9 and FIG. 10, different viewing angles of a schematic diagram of an entire structure of a diseased part supporting assembly of a supporting device according to another embodiment of the disclosure are shown in FIG. 9 and FIG. 10 respectively. A diseased part supporting assembly 500' includes a locking member 503' arranged on a second supporting member 502'. The locking member 503' includes a driving portion 504', a pressing portion 505', and a protrusion 506'. The driving portion 504' is used for driving the locking member 503' to move between a locking location and a releasing location. After the driving portion 504' is pressed, the protrusion 506' arranged on an opposite side of the driving portion 504' is unlocked by taking the pressing portion 505' as a rotation axis such that a location of the diseased part supporting assembly 500' can be adjusted. After adjustment is performed in place, a press force applied to the driving portion 504' is removed, and the protrusion 506' arranged on the opposite side of the driving portion 504' is returned to be locked by still taking the pressing portion 505' as a rotation axis. In this process, the locking member 503' is pressed towards a locking direction. The diseased part supporting assembly 500' further includes a snap-fitting member 513 arranged above the second supporting member 502'. Preferably, the snap-fitting member 513 is arranged between the first supporting member 501' and the second supporting member 502'. Moreover, a gap is provided between the second supporting member 502' and the snap-fitting member 513. Thus, the second supporting member 502' can be more reliably mounted to the third mounting groove 103 and more firmly assembled to the main plate 100. A capacity to bear a diseased part by the first supporting member 501' protruding from a side surface 100b of the main plate 100 is further improved. Preferably, a snap-fitting surface of the snap-fitting member 513 is wider than a flat surface of the second supporting member 502'. The snap-fitting member 513 may be set to include a partial flat surface, or may be set as a clamping structure. It can be easily understood that the snap-fitting member 513 may be arranged in another embodiment to play a same role.

In some embodiments, preferably, the driving portion 504' is preferably rod-shaped or block-shaped. Advantageously, the locking member 503' may be designed to be more flat. A limiting structure (not shown in the figure) such as a recess or a guide rail is arranged on a lower side of the second supporting member 502'. The driving portion 504' can be completely accommodated in the limiting structure. With reference to FIG. 9 and FIG. 10, main bodies of the driving portion 504' and the pressing portion 505' at one end of the locking member 503' and a body of the protrusion 506' at the other end of the locking member 503' are basically located in one plane. Or an included angle delimited by the driving portion, the pressing portion, and the protrusion is not greater than 20°. Such a design saves space and makes the entire structure more compact and concise.

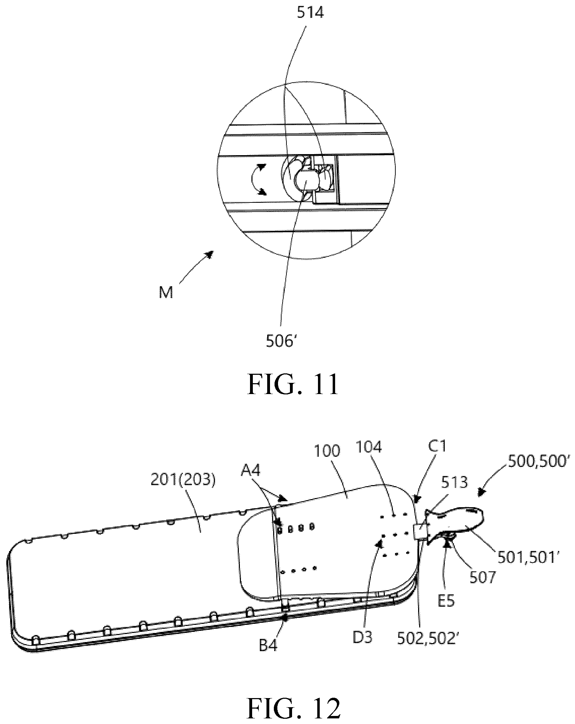

With reference to FIG. 11, an enlarged view of a structure of portion M in FIG. 9 according to another embodiment of the disclosure, which is specifically a schematic structural diagram showing that the protrusion of the locking member and a travel limiting member 514 of the second supporting member match each other, is shown in FIG. 11. The protrusion 506' of the locking member 503' is moved with the pressed driving portion 504' and based on the pressing portion 505' (with reference to a direction indicated by an arrow in FIG. 11), such that the locking member 503' is entirely moved between a locking location and a releasing location. Preferably, the second supporting member 502' further includes the travel limiting member 514. Displacement of the protrusion 506' is limited in at least one direction by the travel limiting member 514. Specifically, the travel limiting member 514 may limit a location of the protrusion 506' in a path in a direction parallel to an extension direction of the second supporting member 502', or may limit a location of the protrusion 506' in a path in a direction perpendicular to an extension direction of the second supporting member 502'. Through such a design, the locking member 503' can be effectively moved in a controllable range. By limiting travel of the protrusion 506' at one end of the locking member 503' in at least one direction, in preferred embodiments, an operation by the locking member 503 flatly designed is more reliable. Excessive adjustment on the protrusion 506' at one end of the locking member 503' is reduced, and possibilities of structural failure and a misoperation by personnel are reduced.

With reference to FIG. 12, a schematic diagram showing that a diseased part supporting assembly is mounted to a main plate on a loading table according to another embodiment of the disclosure is shown in FIG. 12. In combination with FIG. 4 and FIG. 6, when a diseased part supporting assembly 500' is mounted to a main plate 100, a locking hole 104 matching a protrusion 506' is provided on a main plate 100. In a locking location, a locking member 503' is arranged in a third mounting groove 103, and the protrusion 506' is accommodated in the locking hole 104, such that a second supporting member 502' is fixed relative to the main plate 100. In a releasing location, the protrusion 506' is separated from the locking hole 104, such that the second supporting member 502' is movable and adjustable relative to the main plate 100. The locking member 503' may be at least partially accommodated in the third mounting groove 103 together with the second supporting member 502'. After left-right adjustment and front-back adjustment on the second supporting member 502' are completed in the third mounting groove 103, the locking member 503' fixes and locks the diseased part supporting assembly 500'. The driving portion 504' is used for adjusting the locations of the locking member 503', that is, adjusting the locking member from the locking location to the releasing location or from the releasing location to the locking location. In addition to determining a movement axis of the locking member 503', the pressing portion 505' further ensures that the locking member 503' is always located at the locking location in a non-operating state. The protrusion 506' is used for locking the second supporting member 502' in combination with the locking hole 104. Further, in combination with FIG. 10 and descriptions thereof, when the second supporting member 502' is optionally mounted in the third mounting groove 103, a contact portion of the third mounting groove 103 is snap-fitted in a gap between the second supporting member 502' and the snap-fitting member 513. The first supporting member 501' is reliably mounted to a side surface 100b of the main plate 100. It can be easily understood that the snap-fitting member 513 may be arranged in another embodiment to play a same role.

With reference to FIG. 6 to FIG. 12 again, the locking hole 104 is preferably provided above the third mounting groove 103. A plurality of locking holes 104 may be provided. The plurality of locking holes 104 are provided at discrete intervals. The plurality of locking holes 104 are arranged on the main plate 100 in the extension direction. After the second supporting member 502, 502' is moved to a location in the third mounting groove 103, no external force is applied to the driving portion 504, 504'. The protrusion 506, 506' can be optionally combined with one locking hole 104 at a suitable location. Thus, the second supporting member 502, 502' is positioned.

In some embodiments, the diseased part supporting assembly 500 further includes a fastening member 507 for connecting the first supporting member 501, 501' to the second supporting member 502, 502'. The fastening member 507 is movably connected to the second supporting member 502, 502, such that the first supporting member 501, 501' is movable relative to the second supporting member 502, 502'. One end of the second supporting member 502, 502' is located between the first supporting member 501, 501' and the fastening member 507. A through hole 508 is provided on the second supporting member 502, 502'. One portion of the fastening member 507 is located on one side of the second supporting member 502, 502'. The other portion of the fastening member 507 passes through the through hole 508 to be rotationally connected to the first supporting member 501, 501'. After the first supporting member 501, 501' is moved and adjusted, the fastening member 507 is used for fixing and locking the first supporting member. Specifically, when the fastening member 507 is rotated to be not attached to the side of the second supporting member 502, 502', the first supporting member 501, 501' can rotate or slide around a central axis at which the fastening member 507 is located such that an angle or a location of the first supporting member 501, 501' can be adjusted. Thus, comfort of a pose when the irradiated body is fixed can be improved. For example, when the first supporting member 501, 501' is rotated to a suitable location, the fastening member 507 is rotated to be attached to a side of the second supporting member 502, 502'. At this time, the first supporting member 501, 501' can be fixed to the second supporting member 502, 502'.

In some embodiments, the through hole 508 may be not just a circular hole. Preferably, the through hole 508 may be a hole having a length, such as a rectangular hole or a kidney-shaped hole. One or more through holes may be provided. In the embodiment, the through hole 508 is preferably a cambered hole. The fastening member 507 may be optionally moved in the through hole 508. When the fastening member 507 is moved in the through hole 508, the first supporting member 501, 501' is also moved. During movement, in addition to making a fixed pose of the irradiated body more suitable and more comfortable, the first supporting member 501, 501' can be adjusted to a location to make the diseased part closer to the exit of the collimator. In this way, a depth and effect of irradiation treatment are extremely improved while it is ensured that the diseased part can be accurately treated. Specifically, a location of the fastening member 507 moved in the through hole 508 can be identified by marking a scale or by providing holes in communication with each other at equal intervals in a kidney-shaped hole.

In some embodiments, the diseased part supporting assembly 500 further includes at least one adjusting member 509. The adjusting member 509 is used for compensating for a gap generated when the first supporting member 501 is positioned. The adjusting member 509 may be arranged at a lower portion of the first supporting member 501, 501', or may be arranged at a lower portion of the second supporting member 502, 502'. Preferably, a mounting plate 510 is arranged at the lower portion of the second supporting member 502, 502'. Two sides of the mounting plate 510 are rotationally connected to the adjusting member 509. According to a location of the first supporting member 501, 501', the adjusting member 509 is rotated to be extended or contracted, to compensate for a height below the first supporting member 501, 501'. Thus, stable support by the first supporting member 501, 501' can be ensured. For example, when the main plate 100 is arranged on the treatment loading table 201, a gap is provided between the diseased part supporting assembly 500 and the treatment loading table 201. The adjusting member 509 can be extended to compensate for the gap. For another example, when the main plate 100 is arranged on the image loading table 202 having a curved surface, the diseased part supporting assembly 500 is adjusted in a left-right direction. At least two adjusting members 509 are used to compensate for different height differences between the diseased part supporting assembly 500 and the loading table such that the diseased part can be more steadily supported. This is especially applicable to a diseased part having a great weight.

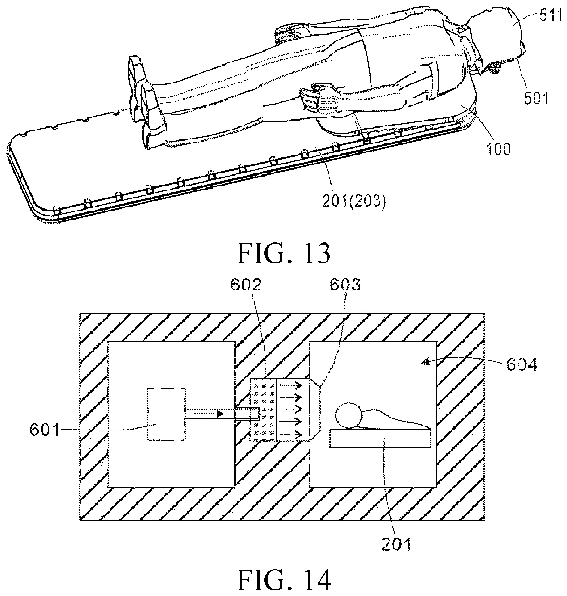

With reference to FIG. 13, a schematic diagram showing that a shape adapting member of a supporting device is wrapped around a diseased part of an irradiated body according to an embodiment of the disclosure is shown in FIG. 13. A diseased part supporting assembly 500 further includes a shape adapting member 511 and fixing members (not shown in the figure). The shape adapting member 511 is used for adapting to a shape of the diseased part of the irradiated body, and includes a hollow structure for a ray to pass through. The fixing members are used for fixing and/or detaching the shape adapting member 511. The shape adapting member 511 has functions of cooling and modeling. The shape adapting member 511 is used for making a mark for a diseased part. When the shape adapting member 511 is heated to a predetermined temperature, such as 70°C, the shape adapting member 511 is wrapped around a surface of the diseased part, such as a head. Subsequently, the shape adapting member 511 is manually pressed to conform to a shape of the head. After the shape adapting member 511 is cooled, modeling of the head of the irradiated body can be implemented. Subsequently, according to a location of the diseased part, a same location on the shape adapting member 511 corresponding to the location of the diseased part is marked. The mark is used for completing irradiation treatment by a beam in an irradiation room. The fixing members are used for fixing the shape adapting member 511 to the first supporting member 501. Several assembly holes 512 (in combination with FIG. 8) are provided on the first supporting member 501. Preferably, the fixing members can be connected to the assembly holes 512 on the first supporting member 501 through a method such as plug in, rotation, or snap fit. Further, the shape adapting member 511 is locked to the first supporting member 501 by the fixing members. Preferably, the shape adapting member 511 is a thermoplastic film. The fixing members are bolts. The assembly holes 512 are threaded holes. Specifically, after the thermoplastic film is wrapped around the head, an edge of the thermoplastic film covers the threaded holes. The thermoplastic film is fixed by screwing the bolts into the threaded holes.

In some embodiments, the first supporting member 501, 501' and the second supporting member 502, 502' of the diseased part supporting assembly 500, 500' may be integrally arranged and are arranged through personalized customization according to the irradiated body.

With reference to FIG. 5 again, during actual use of the supporting device, image scanning is required to be performed on the diseased part. During the scanning, a relative location of the irradiated body is determined, and a location of the diseased part relative to a neutron generating device during irradiation treatment is determined. The main plate 100 is positioned to the image loading table 202 by the second adapting member 401. The diseased part supporting assembly is mounted to the main plate 100. A location of the diseased part supporting assembly is adjusted such that image scanning can be performed on the diseased part.

With reference to FIG. 14, a schematic diagram showing that an irradiated body in a supporting device is subjected to irradiation treatment in an irradiation treatment room according to an embodiment of the disclosure is shown in FIG. 14. Generally, after image scanning, the irradiated body is transferred to an irradiation treatment room 604 or a simulated positioning room (not shown). A main plate 100 is positioned to a treatment loading table 201 by a first adapting member 301. A diseased part supporting assembly is mounted to the main plate 100, and a location of the diseased part supporting assembly is adjusted. Finally, irradiation treatment is performed on a diseased part by a beam generating device 601.

Further, in some embodiments, before the irradiation treatment, simulated positioning may be performed on an irradiated body and a diseased part thereof in the simulated positioning room and a location parameter (described in detail below) obtained after the positioning is recorded. Thus, after being transferred to the irradiation treatment room 604, the irradiated body can be rapidly positioned to a location the same as that in the simulated positioning room according to the location parameter. Moreover, operation time before the irradiation treatment can be reduced. Similarly, used positioning methods of recording a location parameter during previous positioning and rapidly and accurately positioning an irradiated body again in different scenes and/or different time periods are basically the same.

Particularly, the main plate 100 is not limited to being used for image scanning and treatment of a diseased part, is further applicable to another scene requiring positioning, and can adapt to loading tables and/or bearing devices in different shapes in different scenes or in different treatment phases. The main plate is detachably connected to the loading table by an adapting assembly. Detachment includes rapid detachment without requiring a tool and detachment requiring a tool.

In some embodiments, the main plate may be non-detachably arranged on the loading table or may be integrally arranged with the loading table.

An embodiment of the disclosure provides a radiotherapy system. The system includes a beam generating device 601, a loading table, and a supporting device. The beam generating device 601 is used for generating a treatment beam. The loading table is used for at least partially bearing an irradiated body. The supporting device is used for at least partially bearing a diseased part of an irradiated body. The supporting device is detachably connected to the loading table. The supporting device includes a main plate 100 and an adapting assembly. The main plate 100 is detachably connected to the bearing device by the adapting assembly. The adapting assembly is arranged between the main plate 100 and the bearing device 201, 203. The adapting assembly includes a first adapting member 301, a limiting member 302, and a positioning member 303. The first adapting member 301 is adjustably mounted to the main plate 100. The limiting member 302 is adjustably mounted to the main plate 100. The positioning member 303 is adjustably mounted to the bearing device 201, 203, such that the main plate 100 is positioned to the bearing device 201, 203. The beam generating device 601 is preferably a radiation source for generating a neutron of a neutron generating device. Preferably, the neutron generating device includes an accelerator. A charged particle beam generated by the accelerator passes through a beam shaper 602 and a collimator 603 in sequence to irradiate an irradiated body on the loading table. According to requirements in different scenes or in different treatment phases, the supporting device can be positioned to different loading tables. A preparation can be made for irradiation treatment in advance, to ensure that beam treatment is successfully performed.

In some embodiments, the loading table includes a treatment loading table 201 and an image loading table 202. The treatment loading table 201 is used for at least bearing the irradiated body during the irradiation treatment. Subsequently, the irradiation treatment is performed on a diseased part by the beam generating device 601. The image loading table 202 is used for at least bearing the irradiated body during image acquisition. The supporting device includes the main plate 100 and the adapting assembly. The main plate 100 is detachably connected to the treatment loading table 201 or the image loading table 202 by the adapting assembly. The treatment loading table 201 and the image loading table 202 are positioned in two different scenes respectively. The treatment loading table 201 and the image loading table 202 are both used for supporting the irradiated body such that related treatment and preparation work can be made for the irradiated body in respective scenes. The adapting assembly is used for positioning the main plate 100 to a corresponding loading table.

With reference to FIG. 14, the radiotherapy system further includes an irradiation positioning device. The irradiation positioning device is used for performing irradiation positioning on the irradiated body. The treatment loading table 201 is arranged in the irradiation positioning device and used for at least bearing the irradiated body during the irradiation positioning on the irradiated body. The irradiation positioning device is arranged in the irradiation treatment room 604. The main plate 100 is positioned to the treatment loading table 201 by the first adapting member 301. The treatment loading table 201 and the irradiated body on the treatment loading table 201 are supported by the irradiation positioning device. A control device is connected to the irradiation positioning device, and can control an irradiation treatment device to position the irradiated body and a diseased part thereof to perform the irradiation treatment on the diseased part. In the embodiment, structures of the treatment loading table 201 and the first adapting member 301 are the same as those described above, and will not be repeated herein.

With reference to FIG. 5, the radiotherapy system further includes a medical image device (not shown). The medical image device is used for acquiring medical image data of the irradiated body. The image loading table 202 is arranged in the medical image device and used for at least bearing the irradiated body when the medical image data of the irradiated body is acquired. The medical image device is arranged in an image room, and specifically includes a CT device, an MRI device, etc. The main plate 100 is positioned to the image loading table 202 by the second adapting member 401. Medical image data of the irradiated body is obtained by the medical image device, and a mark can be made for a location of the diseased part of the irradiated body. A treatment planning device performs dose simulation computation and generates a treatment plan according to a parameter of a treatment neutron beam generated by the neutron generating device and the medical image data of the diseased part of the irradiated body. The treatment plan can determine a location of the diseased part relative to the neutron generating device during irradiation treatment and corresponding irradiation time. Thus, simulated positioning by using a beam can be performed on the diseased part in the simulated positioning room. In the embodiment, descriptions of the image loading table 202 and the second adapting member 401 are the same as the descriptions about positioning structures during CT and MRI, and will not be repeated herein. In some embodiments, when the irradiated body is required to be moved to switch to different loading tables or irradiation treatment or other preparation work is required to be performed in different phases (for example, interval time is greater than or equal to 12 hours), a worker is only required to mount and position the main plate and/or the diseased part supporting assembly of the supporting device. Through adjustment and fixation, it can be still ensured that after an interval time period, the irradiated body maintains a predetermined pose when being positioned again and is not affected by movement and adjustment in the interval time period. Further, the irradiated body can be separated from the supporting device during movement in different phases or in a same phase. That is, the movement of the irradiated body cannot be limited by the main plate. The irradiated body itself can be transferred to different scenes. Moreover, discomfort caused by an additional constraint and burden for bearing the main plate, etc. cannot occur. In the radiotherapy system, the treatment plan includes important parameters related to treatment, such as a distance between the diseased part and a beam exit, and posture data of the irradiated body. A posture of the irradiated body is limited by the supporting device on different loading tables such that execution of the treatment plan can be effectively ensured.

**In** some embodiments, the loading table further includes a simulation loading table 203. The simulation loading table 203 is used for at least bearing the irradiated body during non-irradiation treatment. The main plate 100 is detachably connected to the simulation loading table 203 by the adapting assembly. Preferably, a shape and structure of the simulation loading table 203 are basically the same as those of the treatment loading table 201. Thus, the main plate 100 can be positioned to the simulation loading table 203 by the first adapting member 301.

With reference to FIG. 4, the radiotherapy system further includes a simulated positioning device (not shown) arranged in the simulated positioning room. The simulated positioning device is used for performing simulated positioning on the irradiated body. The simulation loading table 203 is arranged in the simulated positioning device and used for at least bearing the irradiated body during the simulated positioning on the irradiated body. The simulated positioning device is arranged in the simulated positioning room. The main plate 100 is positioned to the simulation loading table 203 by the first adapting member 301. The simulation loading table 203 and the irradiated body on the simulation loading table 203 are supported by the simulated positioning device. The control device is connected to the simulated positioning device and can control the simulated positioning device. In the embodiment, a main structure of the simulated positioning device in the simulated positioning room and a main structure of the irradiation treatment device in the irradiation treatment room 604 are basically the same, and structures and location relationships of main components are basically the same. For example, the simulation loading table 203 and the treatment loading table 201 have a same width, and include a plurality of similar adapting structures to match the first adapting member 301 together. A related location parameter of the supporting device positioned to the simulation loading table 203 is recorded in the simulated positioning room. Subsequently, when the irradiated body is positioned again in the irradiation treatment room 604 at a different phase, the irradiated body and the diseased part thereof are positioned on the treatment loading table 201 according to the location parameter recorded in the simulated positioning device. Thus, the irradiation treatment is more convenient, efficient, rapid, and accurate.

In some embodiments, the treatment loading table 201 may be further used for simulated positioning.

With reference to FIG. 4 and FIG. 5, a shape of the treatment loading table 201 is different from that of the image loading table 202. A shape of the treatment loading table 201 is the same as that of the simulation loading table 203. Specifically, a shape of a loading surface of the treatment loading table 201 is different from that of a loading surface of the image loading table 202. The treatment loading table 201 and the simulation loading table 203 are flat-surface-shaped. The image loading table 202 is cambered-surface-shaped. During image scanning, the main plate 100 is positioned to the image loading table 202 by the second adapting member 401. During irradiation treatment, the main plate 100 is positioned to the treatment loading table 201 by the first adapting member 301. During simulated positioning, the main plate 100 is positioned to the simulation loading table 203 by the first adapting member 301. Preferably, after being subjected to the image scanning, the irradiated body may be directly subjected to the irradiation treatment. After being subjected to the image scanning, the irradiated body may be first subjected to the simulated positioning, and then subjected to the irradiation treatment. A treatment procedure is selected according to an actual treatment requirement. This is not specifically limited herein. In addition, the image scanning, the simulated positioning, or the irradiation treatment may be performed separately.

In some embodiments, for example, if time of one-time treatment is less than 12 hours, the irradiated body may be entirely transferred when some scenes are switched. No additional positioning and adjustment is required. It can be easily understood that a treatment loading table 201 may be a simulation loading table 203. That is, a same loading table is used in both the simulated positioning room and the irradiation treatment room.

In some embodiments, after the main plate 100 is positioned to the bearing device (the treatment loading table 201 and/or the image loading table 202 and/or the simulation loading table 203) by the first adapting assembly and/or the second adapting member, the diseased part supporting assembly 500 is positioned to the bearing device or the main plate 100 thereof. Moreover, a location of the diseased part supporting assembly is adjusted to support the diseased part of the irradiated body. In the embodiment, structures of the first adapting member 301, the second adapting member 401, and the diseased part supporting assembly 500 are the same as the structures described above, and will not be repeated herein.

An embodiment of the disclosure provides a method for positioning a supporting device. The method includes:
with reference to FIG. 1 to FIG. 4, S100: a supporting device is positioned to a bearing device such as a treatment loading table 201, the supporting device includes a main plate 100 and a first adapting member 301, the first adapting member 301 is mounted to a back surface 100c and/or a side surface 100b of the main plate 100, and the main plate 100 is connected to a predetermined location on the treatment loading table 201 by the first adapting member 301. This step is used for being implemented when irradiation treatment is performed in an irradiation treatment room 604. It can be easily understood that the back surface or the side surface of the main plate may have a boundary line or may have no clear boundary line. In some embodiments, the supporting surface of the main plate may smoothly transition to the back surface of the main plate. When the irradiated body is required to be moved for scene switching or is required to be supported and positioned in different phases, by mounting and positioning the supporting device, it can be still ensured that the irradiated body maintains a predetermined pose when being positioned again and is not affected by movement and adjustment of the irradiated body. The irradiated body can be separated from the supporting device during movement such that no additional constraint and burden exist.

Specifically, S101: At least two first mounting structures are arranged on the back surface 100c and/or the side surface 100b of the main plate 100, the first mounting structure includes a first mounting groove 101a and a mounting hole 101b, and according to a location of the main plate 100, a matching mounting location of the first adapting member 301 relative to the first mounting groove 101a and the mounting hole 101b is selected for mounting. S102: At least two adapting structures for mounting the first adapting members 301 are arranged on the treatment loading table 201, the adapting structure includes an adapting groove 201a, and according to the location of the main plate 100, a matching mounting location of the first adapting member 301 relative to the adapting groove 201a is selected for mounting. S103: A first supporting member 501 and a second supporting member 502 are positioned to the main plate 100, at least one third mounting structure capable of accommodating the second supporting member 502 is arranged on the main plate 100, the third mounting structure includes a third mounting groove 103, and according to a location of a diseased part of an irradiated body, a matching mounting location of the second supporting member 502 relative to a beam generating device 601 is selected for mounting. S104: At least two fourth mounting structures for locking the second supporting member 502 are arranged on the main plate 100, the fourth mounting structure includes a locking hole 104, and according to the location of the diseased part of the irradiated body, a matching mounting location of the second supporting member 502 relative to the locking hole 104 is selected for mounting. S105: A fastening member 507 for locking the first supporting member 501 is arranged on the second supporting member 502, and according to the location of the diseased part of the irradiated body, a matching mounting location of the first supporting member 501 relative to the second supporting member 502 is selected for mounting. After the supporting device is positioned to the treatment loading table 201, irradiation treatment can be performed on the diseased part. In S101 to S105 mentioned above, a mounting sequence is not limited, and one or more steps can be implemented according to actual treatment situations.

In some embodiments, in the above steps, the treatment loading table 201 may be replaced with a simulation loading table 203. Methods of positioning the supporting device to the simulation loading table 203 are basically the same.

With reference to FIG. 5, S200: the supporting device is positioned to the bearing device such as an image loading table 202, the supporting device further includes a second adapting member 401, the second adapting member 401 is mounted to the back surface 100c and/or the side surface 100b of the main plate 100, and the main plate 100 is connected to a predetermined location on the image loading table 202 by the second adapting member 401. This step is implemented when image scanning is performed in an image scanning room.

Specifically, S201: At least two second mounting structures are arranged on the back surface 100c and/or the side surface 100b of the main plate 100, the second mounting structure includes a second mounting groove 102, a matching mounting location relative to the image loading table 202 is selected according to a width of the image loading table 202, a second adapting member 401 is mounted in the second mounting groove 102, and according to the location of the main plate 100, a matching mounting location relative to the image loading table 202 is selected for mounting. S202: An adapting surface 401a is arranged on the second adapting member 401, the adapting surface 401a is snap-fitted to a surface and/or an edge of the image loading table 202, such that the main plate 100 is limited relative to the image loading table 202.

In some embodiments, S300: the supporting device is detached from the image loading table 202, the second adapting member 401 is detached from the main plate 100, and a first adapting member 301 is mounted. Or, the supporting device is detached from the treatment loading table 201, the first adapting member 301 is detached from the main plate 100, and the second adapting member 401 is mounted. This step is implemented when the loading table is switched and interference occurs.

Specifically, when the image loading table 202 is switched to the treatment loading table 201, the second adapting member 401 is detached from the second mounting groove 102, and the first adapting member 301 is mounted to the first mounting groove 101a such that irradiation treatment can be performed. When the treatment loading table 201 is switched to the image loading table 202, the first adapting member is detached from the first mounting groove 101a, and the second adapting member 401 is mounted to the second mounting groove 102 such that medical scanning can be performed. When the loading table is switched, if no mounting or location interference occurs on different adapting assemblies, detachment may not be performed. It can be easily understood that the treatment loading table 201 in the above method may be replaced with a simulation loading table 203.

With reference to FIG. 4 to FIG. 6, S400: when the irradiated body is positioned to the bearing device such as a treatment loading table or a simulation loading table, by positioning the supporting device to the treatment loading table 201 or the simulation loading table 203, a parameter of the main plate 100 of the supporting device and a location parameter of the supporting device on the treatment loading table 201 or the simulation loading table 203 are recorded. Subsequently, before the irradiated body is transferred to the treatment loading table 201 or the simulation loading table 203 again, the main plate 100 is positioned to the treatment loading table 201 or the simulation loading table 203 based on the location parameter. In some embodiments, the location parameter includes a first location parameter. At least two first mounting structures are arranged on the back surface 100c and/or the side surface 100b of the main plate 100. The first mounting structure includes a first mounting groove 101a and a mounting hole 101b. According to a location of a diseased part of the irradiated body and a location of the main plate 100, a matching mounting location relative to the first mounting groove 101a and the mounting hole 101b is selected. A first adapting member is assembled in the first mounting structure. First location parameters A, that is, location numbers (101a1, 101a2, 101a3, or 101a4, etc.; and 101b1, 101b2, 101b3, or 101b4, etc.) of the selected first mounting groove 101a and mounting hole 101b that are selected are recorded. This step is generally used for being implemented when simulated positioning is performed in a simulated positioning room or an irradiation treatment room.

In some embodiments, the location parameter includes a second location parameter. S401: At least two adapting structures for mounting the first adapting members 301 are arranged on the treatment loading table 201 or the simulation loading table 203, the adapting structure includes an adapting groove 201a, a matching mounting location relative to the adapting groove 201a is selected according to the location of the diseased part of the irradiated body and the location of the main plate 100, the main plate is assembled in the adapting structure by the first adapting member 301, and a second location parameter B, advantageously, a location number (201a1, 201a2, 201a3, 201a4, or 201a5, etc.) of the adapting groove 201a that may be selected is recorded.

With reference to FIG. 6 to FIG. 13, S500: a diseased part supporting assembly 500 is positioned to the main plate 100 of the treatment loading table 201 or the simulation loading table 203, and when the irradiated body is positioned to the diseased part supporting assembly 500 and the treatment loading table 201 or the simulation loading table 203, a location parameter of the diseased part supporting assembly 500 and a location parameter of the diseased part supporting assembly on the treatment loading table 201 or the simulation loading table 203 are recorded. Before the irradiated body is transferred to the diseased part supporting assembly 500 and the treatment loading table 201 or the simulation loading table 203 again, the diseased part supporting assembly 500 is first positioned to the treatment loading table 201 or the simulation loading table 203 based on the location parameter. In some embodiments, the location parameter includes a third location parameter. The diseased part supporting assembly 500 includes a first supporting member 501 protruding from a side surface 100b of the main plate 100 and a second supporting member 502. The first supporting member 501 is connected to the main plate 100 by the second supporting member 502. At least one third mounting structure capable of accommodating the second supporting member 502 is arranged on the main plate 100. The third mounting structure includes a third mounting groove 103. A matching mounting location relative to the beam generating device 601 and the third mounting structure is selected based on the location of the diseased part of the irradiated body. The second supporting member is assembled in the third mounting structure. A third location parameter C, advantageously, a location number (103-1, 103-2, 103-3, 103-4, or 103-5, etc.) of the third mounting groove 103 that may be selected is recorded.

In some embodiments, the location parameter includes a fourth location parameter. S501: At least two fourth mounting structures for locking the second supporting member 502 are arranged on the main plate 100, the fourth mounting structure includes a locking hole 104, a matching mounting location relative to a structure of the locking hole 104 is selected according to the location of the diseased part of the irradiated body, the second supporting member is assembled in the fourth mounting structure, and a fourth location parameter D, that is, a location number (104-1, 104-2, 104-3, 104-4, or 104-5, etc.) of the selected locking hole 104, which is equal to a mounting depth of the second supporting member 502, is recorded.

In some embodiments, the location parameter includes a fifth location parameter. S502: A fastening member 507 for adjusting and locking the first supporting member 501 is arranged on the second supporting member 502, according to the location of the diseased part of the irradiated body, a matching mounting location of the first supporting member 501 relative to the second supporting member 502 is selected for mounting, and a fifth location parameter E, that is, location data (501-1, 501-2, 501-3, 501-4, or 501-5, etc.) of slide or rotation of the first supporting member 501 relative to the second supporting member 502 is recorded.

In some embodiments, when the irradiated body is positioned again, the main plate may be positioned to the treatment loading table or the simulation loading table based on a group of location parameters. S600: When the simulation loading table 203 is switched to the treatment loading table 201 for the irradiated body, or a patient is required to be positioned to the treatment loading table 201 or the simulation loading table 203 when being treated again, the main plate 100 is positioned to the treatment loading table 201 or the simulation loading table 203 based on the first location parameter and the second location parameter that are recorded. In an embodiment, when the simulation loading table 203 is switched to the treatment loading table 201, that is, when an irradiated body is transferred from a simulated positioning room to an irradiation treatment room 604, the first location parameter and the second location parameter that are the same as those in the simulated positioning room are selected in the irradiation treatment room 604, such that locations of the main plate 100 relative to the simulation loading table 203 and the treatment loading table 201 are the same. Thus, the locations of the irradiated body in two scenes are also kept the same. It can be easily understood that the above group of location parameters may include the first location parameter, the second location parameter, etc.

In some embodiments, when the irradiated body is positioned again, the diseased part supporting assembly may be positioned to the bearing device or the main plate thereof based on another group of location parameters. S700: The diseased part supporting assembly is positioned to the main plate 100 based on the third location parameter, the fourth location parameter, and the fifth location parameter. When the loading table is switched, for example, when the simulation loading table 203 is switched to the treatment loading table 201, or when the image loading table 202 is switched to the treatment loading table 201/the simulation loading table 203, the third location parameter, the fourth location parameter, and the fifth location parameter that are the same as those in the medical image room/the simulated positioning room (not shown) are selected in the irradiation treatment room 604/the simulated positioning room (not shown), such that locations of the diseased part supporting assembly 500 relative to the main plate 100 are the same, and locations of the diseased part of the irradiated body in two scenes are also kept as same as possible. Thus, precise irradiation by a beam in the irradiation treatment room 604 is facilitated. The above location parameters may be further applied to location determination when a patient is treated again. It can be easily understood that the another location parameter may include the third location parameter and/or the fourth location parameter and/or the fifth location parameter, etc.

In some embodiments, in the above steps, the treatment loading table 201 or the simulation loading table 203 may be replaced with the image loading table 202. Methods of positioning the supporting device to the image loading table 202 and acquiring a location parameter are basically the same.

In some embodiments, before the irradiated body is transferred to the irradiation treatment room 604, if simulated positioning is required to be performed on the irradiated body and the diseased part thereof, that is, the irradiated body is transferred from the image scanning room to the simulated positioning room, the main plate 100 is positioned to the simulation loading table 203 in the simulated positioning room by the first adapting member 301. The diseased part supporting assembly is mounted to the main plate 100 and adjusted. Thus, simulated positioning is performed on the irradiated body and the diseased part thereof. A current location of the irradiated body positioned in the simulated positioning room is recorded. Preferably, a plurality of mounting holes 101b and a plurality of third mounting grooves 103 are provided on the main plate 100. In a case that a plurality of adapting grooves 201a are provided on the simulation loading table 203, selectable locations of the plurality of mounting holes 101b are defined as an array A. Selectable locations of the plurality of adapting grooves 201a are defined as an array B. Selectable locations of the second supporting member 502 relative to the third mounting groove 103 are defined as an array C. Selectable locations of the plurality of locking holes 104 are defined as an array D. Selectable locations of the first supporting member 501 sliding or rotating relative to the second supporting member 502 are defined as an array E. The selectable locations of the arrays A, B, C, D, and E mentioned above are numbered, such as a selected location A4 of the limiting member 302 relative to the mounting hole 101b, a selected location 201a4 on the adapting groove 201a, which is referred to as B4 for short in the embodiment, of the first adapting member 301 relative to the simulation loading table 203, a selected location (including a specifically selected location of a third mounting groove 103-1, which is referred to as C1 for short; and a selected location 104-3 on the locking hole 104, which is referred to as D3 for short, of a protrusion 506 when the second supporting member is positioned in the third mounting groove 103) of the second supporting member 502 relative to the third mounting groove 103, and a location (including an angle by which the second supporting member 502 is rotated, and location data 501-5, which is referred to as E5 for short, of movement of the fastening member 507 in a through hole 508) of the first supporting member 501 relative to the second supporting member 502. A sequence of the location parameters may not be limited. After being subjected to the simulated positioning in the simulated positioning room, the irradiated body is transferred to a treatment room. A location parameter (A4-B4-C1-D3-E5) the same as that in the simulated positioning room is selected, such that positioning of the diseased part of the irradiated body in two scenes and/or in a same scene in different time periods is kept as consistent as possible. In this way, time for positioning the irradiated body in the treatment room can be reduced. Comfort of the irradiated body is improved, such that the irradiated body is prevented from suffering from an unnecessary constraint and limitation. Moreover, the irradiated body is prevented from being unnecessarily irradiated by particles.

In some embodiments, optionally, location parameters recorded when the irradiated body is transferred from the image scanning room may be the third location parameter, the fourth location parameter, and the fifth location parameter. Or, the location parameter applicable to the irradiated body and recorded in a same scene may be the third location parameter, the fourth location parameter, and the fifth location parameter. Subsequently, the recorded location parameter may be used in a same scene or different scenes in different time periods.

One or more steps of the above method for positioning a supporting device may be implemented according to an actual treatment situation, and specific sequences of mounting and operation are not limited.

Technical features of the above embodiments can be combined with one another. To make description concise, not all possible combinations of the technical features in the above embodiments are described. However, the combinations of these technical features should be considered as falling within the scope recited in the description provided that no conflict exists.

The above embodiments express merely some types of implementations of the disclosure, and are specifically described in details, but cannot be interpreted as limiting the scope of patent for the disclosure as a result. It should be noted that a person of ordinary skill in the art can further make several variations and improvements without departing from the conception of the disclosure. These variations and improvements all fall within the scope of protection of the disclosure. Thus, the scope of protection of the patent for the disclosure should be subjected to the appended claims.

## Claims

1. A supporting device, comprising:
a main plate used for at least partially bearing an irradiated body, wherein the main plate defines an extension direction, and the main plate comprises a supporting surface arranged in the extension direction, a side surface connected to the supporting surface, and a back surface arranged opposite the supporting surface; and
an adapting assembly arranged on the back surface and/or the side surface of the main plate and used for connecting the main plate to a predetermined location.

2. The supporting device according to claim 1, wherein the main plate is detachably connected to the predetermined location on a bearing device by the adapting assembly, the main plate comprises at least one mounting structure for mounting the adapting assembly, and the adapting assembly is adjustably mounted in the mounting structure.

3. The supporting device according to claim 2, wherein the bearing device comprises at least one adapting structure for mounting the adapting assembly, and the adapting assembly matches the adapting structure, so as to limit the main plate relative to the bearing device.

4. The supporting device according to claim 3, wherein the bearing device comprises a treatment loading table, the adapting assembly is arranged between the main plate and the treatment loading table, the adapting assembly comprises a first adapting member, a limiting member, and a positioning member, the limiting member is adjustably mounted to the main plate, the first adapting member is adjustably mounted to the main plate, and the positioning member is adjustably mounted to the treatment loading table, so as to position the main plate to the treatment loading table.

5. The supporting device according to claim 4, wherein the mounting structure comprises a first mounting groove, and the first adapting member is arranged in a direction perpendicular to the extension direction and adjustably mounted in the first mounting groove.

6. The supporting device according to claim 5, wherein the mounting structure further comprises a mounting hole, the first adapting member is at least partially accommodated in the first mounting groove and optionally positioned to the main plate by the limiting member, the limiting member protrudes from the first adapting member, the limiting member is optionally assembled in the mounting hole, the mounting hole corresponds to the first mounting groove in location and is used for matching the limiting member, the adapting structure comprises an adapting groove, the positioning member protrudes from the first adapting member, and the positioning member is optionally assembled in the adapting groove.

7. The supporting device according to claim 2 or 4, wherein the bearing device comprises an image loading table, the adapting assembly is arranged between the main plate and the image loading table, the adapting assembly comprises a second adapting member, the second adapting member comprises an adapting surface, and the adapting surface is adjustably matched to a surface and/or an edge of the image loading table.

8. The supporting device according to claim 7, wherein the mounting structure comprises a second mounting groove, the second adapting member is adjustably mounted in the second mounting groove, and the second adapting member is at least partially accommodated in the second mounting groove, so as to limit the main plate relative to the image loading table at least in the direction perpendicular to the extension direction.

9. A method for positioning a supporting device, comprising:
positioning the supporting device to a bearing device, wherein the supporting device comprises a main plate and a first adapting member; and mounting the first adapting member on a back surface and/or a side surface of the main plate, wherein the main plate is connected to a predetermined location on the bearing device by the first adapting member.

10. The method for positioning a supporting device according to claim 9, comprising:
recording location parameters of the main plate and the bearing device when an irradiated body is positioned to the bearing device; and positioning the main plate to the bearing device based on the location parameters before the irradiated body is transferred to the bearing device again.

11. The method for positioning a supporting device according to claim 9, comprising:
positioning a diseased part supporting assembly to the main plate on the bearing device, and recording location parameters of the diseased part supporting assembly and the bearing device when the irradiated body is positioned to the diseased part supporting assembly and the bearing device; and positioning the diseased part supporting assembly to the bearing device based on the location parameters before the irradiated body is transferred to the diseased part supporting assembly and the bearing device again.

12. A radiotherapy system, comprising:
a beam generating device used for generating a treatment beam;
a bearing device used for bearing an irradiated body; and
a supporting device used for at least partially bearing the irradiated body, wherein the supporting device is detachably connected to the bearing device.

13. The radiotherapy system according to claim 12, wherein the supporting device comprises a main plate and an adapting assembly, and the main plate is detachably connected to the bearing device by the adapting assembly.

14. The radiotherapy system according to claim 12, further comprising: at least one diseased part supporting assembly, wherein the diseased part supporting assembly is used for at least partially supporting a diseased part of the irradiated body, and the diseased part supporting assembly is detachably connected to the bearing device.

15. The radiotherapy system according to claim 14, wherein the bearing device comprises a supporting surface, a side surface connected to the supporting surface, and a back surface arranged opposite the supporting surface, the diseased part supporting assembly comprises a first supporting member at least partially protruding from the side surface and a second supporting member, the first supporting member is detachably connected to the bearing device by the second supporting member, the first supporting member is movable relative to the second supporting member, the second supporting member is movable relative to the bearing device, and the second supporting member is arranged on the side surface and/or the back surface.
